# EUROPEAN PATENT APPLICATION

(11) **EP 2 446 811 A1**
(43) Date of publication of application: **02.05.2012**
(21) Application number: 10791959.9
(22) Date of filing: 07.06.2010
(51) Int. Cl.: A61B 3/08, A61B 3/113

(54) **OPHTHALMIC TEST DEVICE AND HESS SCREEN TEST DEVICE**

(30) Priority: 25.06.2009 JP 2009150887
(71) Applicant: Panasonic Corporation, Osaka 571-8501 (JP)
(72) Inventor: KOIWA, Hiroko, Kadoma-shi Osaka 571-8686 (JP); KAWAMURA, Ryo, Kadoma-shi Osaka 571-8686 (JP)
(74) Representative: Appelt, Christian W.
(86) International application number: PCT/JP2010/059590
(87) International publication number: WO 2010/150641

(57) **Abstract**

An ophthalmic apparatus (1) includes: a screen (11); a examination controller (3); a first image projection unit (5) projecting a target image; a second image projection unit (7) projecting a pointer image; a mirror (9) reflecting the projections of the first and second image projection units (5) and (7) to project the same onto the screen (11); and a pointer operation input unit (13) through which the pointer position is inputted. The examination controller (3) includes: a first image generation unit (21) generating a target image; an image correction unit (22) correcting the target image so that the target image does not look distorted on the screen (11); a calculation storage unit (24) calculating the position of the pointer according to the input through the pointer operation input unit (13) and storing the calculated position; a second image generation unit (23) generating the image of the pointer; and an output unit (25) presenting or outputting the target image and the position of the pointer.

## Description

### [Technical Field]

The invention relates to an ophthalmic apparatus and a Hess test apparatus performing ophthalmic examinations including eye position and eye movement measurement.

### [Background Art]

Impairments of eye movement result in strabismus in which the lines of sight of right and left eyes do not meet at a single point of fixation or diplopia in which an object is seen double, As an examination for such impairments, a Hess red-green test has been used (Non-Patent Literature 1).

In the Hess red-green test, glasses with red and green lenses are individually worn by one and the other eyes of a subject, and a Hess screen with a red grid is shown to the subject. Then, an indicator target of a green spot operated by the subject is caused to sequentially indicate red targets on the screen. The red targets on the screen can be seen by only the eye wearing the green lens, and the green indicator target can be seen by only the eye wearing the red lens. Accordingly, if the subject has strabismus or diplopia, an eye position deviation is caused, which is a difference between the target and each indicator target. The examiner inputs the eye position deviation at an electronic chart input terminal using an input template of the same grid as the Hess screen (for example, an electronic chart input system described in PTL 1).

### [Citation List]

### [Patent Literature]

[PTL 1] Japanese Patent Laid-open Publication No. 2005-218563

### [Non Patent Literature]

[NPL 1] "Modern Ophthalmology" (KANEHARA & CO., Ltd., Revised, 10th edition, 256, April 2009)

### [Summary of Invention]

### [Technical Problem]

However, in the aforementioned electronic chart input system, the Hess screen is projected onto the screen surface using a wide-angle fisheye lens as the previous ones, and the subject undergoes the examination with red-green glasses. Accordingly, the system includes some problems such as: distortion of the grid; the separation of right and left views using the red-green glasses, which is different from the real environment of daily life; and non-equivalent distance between the subject's eye position and each point on the screen surface due to the planar screen.

In the light of the aforementioned problems, an object of the invention is to provide an ophthalmic apparatus which provides an examination environment close to the real environment and allows the subject in the natural condition for a high examination precision.

### [Means to Solve Problems]

In order to solve the aforementioned problems, an aspect of the present invention is an ophthalmic apparatus including: a screen keeping distance constant from a subject's eye position; a first image generation unit generating a target image; an image correction unit correcting the target image generated by the first image generation unit to cause the target image not to look distorted on the screen in the eyes of the subject; a first image projection unit projecting onto the screen, the target image generated by the first image generation unit; a pointer operation input unit through which the subject inputs the positional operation of the pointer; a calculation storage unit calculating the position of the pointer in the target image according to the input through the pointer operation input unit and storing the calculated position; a second image generation unit generating the image of the pointer at the position in the target image calculated by the calculation storage unit; a second image projection unit projecting onto the screen, the image generated by the second image generation unit; and an output unit presenting or outputting the position of the pointer together with the target image.

The ophthalmic apparatus of the present invention can further include an eye position deviation calculation unit which converts the position of the pointer in the target image calculated by the calculation storage unit to a deviation angle with respect to a viewing position of the subject and outputting the deviation angle to the output unit.

The ophthalmic apparatus of the present invention can further include a subject's viewing position input unit through which an examiner inputs the eye position of the subject.

The ophthalmic apparatus of the present invention can further include a subject's viewing position detection unit detecting the eye position of the subject based on an image obtained by capturing an image of the subject.

The ophthalmic apparatus of the present invention can further include a driving unit moving the position of the subject; and a subject position control unit controlling the driving unit based on results of the detection by the subject's viewing position detection unit to operate the position of the subject with respect to the screen.

The ophthalmic apparatus of the present invention can be included in a Hess test apparatus.

### [Advantageous Effects of Invention]

According to the present invention, the target image generated by the first image generation unit is corrected so as not to look distorted on the screen in the eyes of the subject. This has an effect of providing a natural target image without distortion unlike the conventional image of the grid projected from a fisheye lens.

Furthermore, according to the present invention, the image corrected by the image correction unit and the image of the pointer generated by the second image generation unit can be separated into images visible by the right and left eyes using an optical system of polarizers, color filters, a lenticular lens, or the like in addition to the conventional red-green separation method. This has an effect of conducting an ophthalmic examination in a natural condition in which the subject usually lives.

Still furthermore, according to the present invention, the ophthalmic apparatus includes: the pointer operation input unit through which the subject inputs the positional operation of the pointer; the calculation storage unit calculating the position of the pointer on the target image according to the input through the pointer operation input unit and storing the calculated position; and the output unit presenting or outputting the position of the pointer together with the position of the target image. This has an effect of automatically and accurately recording the eye position deviations without causing the examiner to write the eye position deviations to the chart by hand.

### [Brief Description of Drawings]

Fig. 1 is a configuration view explaining Embodiment 1 of an ophthalmic apparatus according to the present invention.
Fig. 2 is a flowchart explaining the operation of Embodiment 1.
Figs. 3A and 3B are views showing examples of examination result chart in Embodiment 1, Fig. 3A showing a target image, Fig. 3B showing a display in which the positions of the pointer specified by the subject are connected by line segments.
Fig. 4 is a configuration view explaining Embodiment 2 of an ophthalmic apparatus according to the present invention.
Fig. 5 is a flowchart explaining the operation of Embodiment 2.
Fig. 6 is a view showing an example of the examination result chart in Embodiment 2.
Fig. 7 is a configuration view explaining Embodiment 3 of the ophthalmic apparatus according to the present invention.
Fig. 8 is a flowchart explaining the operation of Embodiment 3.
Fig. 9 is a configuration view explaining Embodiment 4 of the ophthalmic apparatus according to the present invention.
Fig. 10 is a schematic flowchart explaining the operation of Embodiment 4.
Fig. 11 is a detailed flowchart explaining optimal adjustment of the position of the subject's eye in Embodiment 4.

### [Modes for Carrying out Invention]

Next, a description is given of embodiments of the present invention in detail with reference to the drawings. An ophthalmic apparatus shown in the following embodiments is suitable especially to eye position/eye movement measurements and Hess tests. However, the ophthalmic apparatus of the invention is applicable to binocular function examination and aniseikonia examination.

### [Embodiment 1]

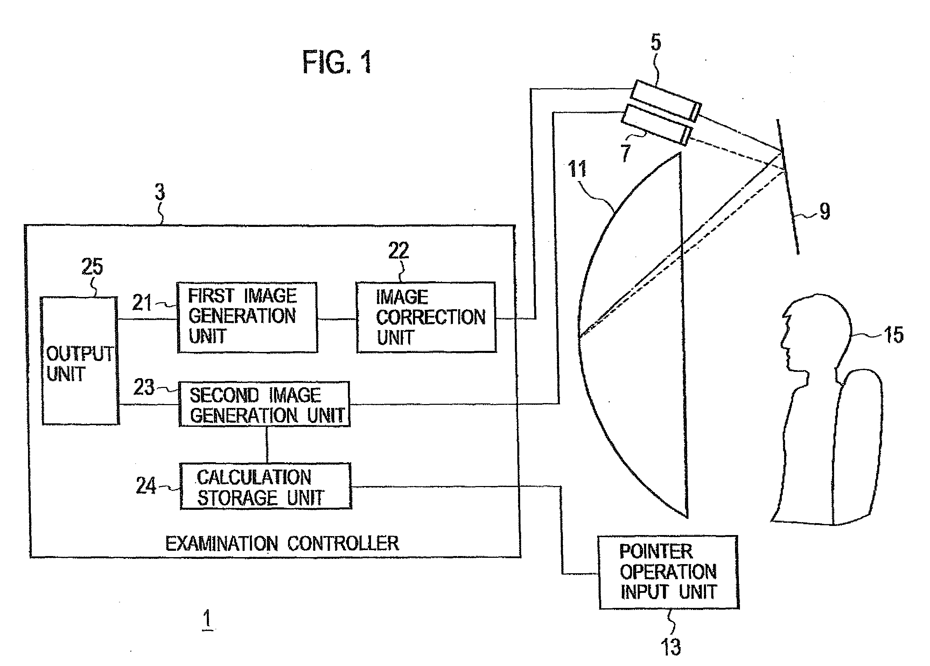

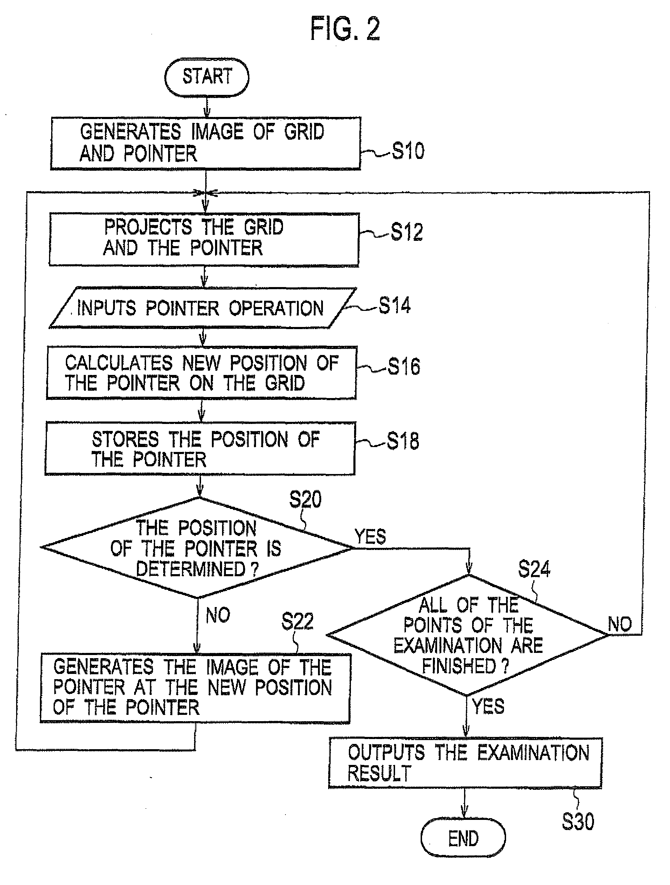

Fig. 1 is a configuration view explaining the configuration of Embodiment 1 of the ophthalmic apparatus according to the present invention. Fig. 2 is a flowchart explaining the operation of Embodiment 1. Figs, 3A and 3B are views showing output examples of a examination result chart in Embodiment 1.

In Fig. 1, the ophthalmic apparatus 1 includes: a screen 11 having such a profile that keeps the distance constant from the eye position of a subject 15; a examination controller 3 generating examination images and controlling the entire examination; a first image projection unit 5 projecting onto the screen 11, a target image corrected by a later-described image correction unit 22 of the examination controller 3; a pointer operation input unit 13 through which the subject 15 inputs a positional operation for a pointer; a second image projection unit 7 projecting onto the screen 11, an image of the pointer generated by a second image generation unit 23 of the examination controller 3; and a mirror 9 reflecting the images projected from the first and second image projection units 5 and 7 to project the same onto the screen 11.

Preferably, the screen 11 has a spherical shape. The screen 11 may be provided with a rest on which the chin of the subject 15 is placed so that the eye position of the subject 15 coincides with the center of the sphere. Preferably, the screen 11 is large enough to cover at least a 20-degree range around the eye position of the subject 15.

The images from the first and second image projection units 5 and 7 can be separately presented to the subject's right and left eyes by (linear or circular) polarizers, color filters, a lenticular lens, or the like.

Moreover, at a Hess test by the ophthalmic apparatus 1, the subject 15 wears polarized glasses, glasses with lenses of color filters, or the like. Accordingly, the image from the first image projection unit 5 is separately presented to only one of the eyes of the subject 15 while the image from the second image projection unit 7 is separately presented to only the other eye.

The examination controller 3 includes: a first image generation unit 21 generating a target image (for example, an image of a grid); the image correction unit 22 correcting the target image generated by the first image generation unit 21 so that the target image does not look distorted on the screen 11 in the eyes of the subject 15 (for example, so that the target image is a square); a calculation storage unit 24 calculating the position of the pointer in the target image according to the input through the pointer operation input unit 13 and storing the calculated position; the second image generation unit 23 generating an image of the pointer at the position in the target image calculated by the calculation storage unit 24; and an output unit 25 presenting or outputting the position of the target image together with the position of the pointer.

The examination controller 3 is composed of a personal computer or a work station as hardware, for example. The examination controller 3 includes a program configured to generate images for the ophthalmic examination and control the generated images and the entire examination, which is installed in the above hardware.

The pointer operation input unit 13 through which the subject 15 inputs the positional operation for the pointer is connected to the examination controller 3. The pointer operation input unit 13 is an input device by which an operation to move the position of the pointer to an arbitrary position on the screen 11 can be inputted, such as a mouse, a trackball, or a joy stick. The pointer operation input unit 13 may be provided with a position determination input to determine the position of the pointer after the pointer is moved. If the pointer operation input unit 13 is a mouse, for example, the position determination input can be defined as a click of the right or left click button of the mouse.

The examination controller 3 includes at least two image signal output ports (not shown). One of the image signal output ports outputs the image signal of the target image from the image correction unit 22 to the first image projection unit 5. The other image signal output port outputs the image signal of the pointer from the second image generation unit 23 to the second image projection unit 7.

The image correction unit 22 corrects the target image generated by the first image generation unit 21 so that the target image on the screen 11 does not look distorted to the subject 15 when the target image is projected from the first image projection unit 5 through the mirror 9 onto the screen 11. Accordingly, it is assumed that the image correction unit 22 stores relational expressions expressing the profiles of the first image projection unit 5, mirror 9, and screen 11 and the positional relation thereof and also stores an algorithm to correct the target image.

The second image generation unit 23 generates an image of an arrow indicating the position of the pointer. If the subject 15 inputs an operation to move the position of the pointer through the pointer operation input unit 13, the calculation storage unit 24 calculates the position of the pointer so that the position of the pointer moves on the screen 11. This calculation result is outputted from the calculation storage unit 24 to the second image generation unit 23. The second image generation unit 23 generates an image with the position of the pointer updated based on the output from the calculation storage unit 24. The subject 15 can thus control the position of the pointer on the screen 11. The subject 15 may input the pointer position determination through the pointer operation input unit 13 when one of the eyes of the subject 15 fixates on the target image on the screen 11 and the position of the pointer seen by the other eye looks superimposed on the point specified in the target image. Alternatively, the pointer operation input unit 13 may determine the position of the pointer when the position of the pointer does not change for a certain period of time or more.

In the case where the pointer operation input unit 13 is provided with the input of pointer position determination, if the pointer operation input unit 13 is a mouse, for example, the input of pointer position determination can be defined as the right click button.

The output unit 25 is a device capable of outputting images to a printer, a liquid crystal display, and the like which output a examination result chart including the target image and the positions of the points in the target image specified by the subject using the pointer and outputting the text information on the position deviations of the both eyes as the examination results to the first and second image projection units.

Next, the operation of the ophthalmic apparatus 1 is described with reference to the flowchart of Fig. 2. At first, in step 10 (hereinafter, the step is abbreviated as S), the examination controller 3 generates the target image with the first image generation unit 21 and corrects the target image with the image correction unit 22 so that the target image does not look distorted in the eyes of the subject 15 when the target image is projected onto the screen 11. At the same time, the second image generation unit 23 generates an image of an arrow or the like as the image of the pointer.

Subsequently, in S12, the first image projection unit 5 projects the corrected target image onto the screen 11, and the second image projection unit 7 projects the image of the pointer onto the screen 11.

The examiner then gives the subject 15 an instruction on which point in the target image projected onto the screen 11 the subject 15 will fixate. While fixating on the instructed point with one of the eyes, the subject 15 operates the pointer operation input unit 13 so that the position of the pointer seen by the other eye is superimposed on the instructed point. The operation signal of the pointer operation input unit 13 is taken into the calculation storage unit 24 in S14.

Subsequently, in S16, the calculation storage unit 24 calculates the direction and distance that the pointer moves and calculates a new position of the pointer in the target image based on the operation signal inputted from the pointer operation input unit 13.

Subsequently, in S 18, the calculation storage unit 24 stores the position of the pointer calculated in S16.

In S20, the pointer operation input unit 13 then determines whether the position of the pointer is determined. This determination is based on whether there is the position determination input if the pointer operation input unit 13 is provided with the input of pointer position determination. If the pointer operation input unit 13 is not provided with the input of pointer position determination, the position is determined when the position of the pointer stays at a certain position for a predetermined time (10 sec, for example) or more. If the position of the pointer is not determined in the determination of S20, the process proceeds to S24.

In S22, the second image generation unit 23 generates the image of the pointer at the new position of the pointer calculated by the calculation storage unit 24 in S16, and the process returns to S12. According to the input of the operation by the subject 15 through the pointer operation input unit 13, the image with the position of the pointer updated is generated, and the target image and the updated pointer are projected.

In S24, it is determined whether all of the points of the examination are finished. In a normal Hess test, for example, the examination is performed at the total nine points including No. 1 at the center, No, 2 at 15 degrees above No. 1, and No. 2 to No. 9 clockwise. If the examination for all the points is not finished, the process returns to S12 for the examination using the new point. If the examination for all the points is finished, the process proceeds to S30.

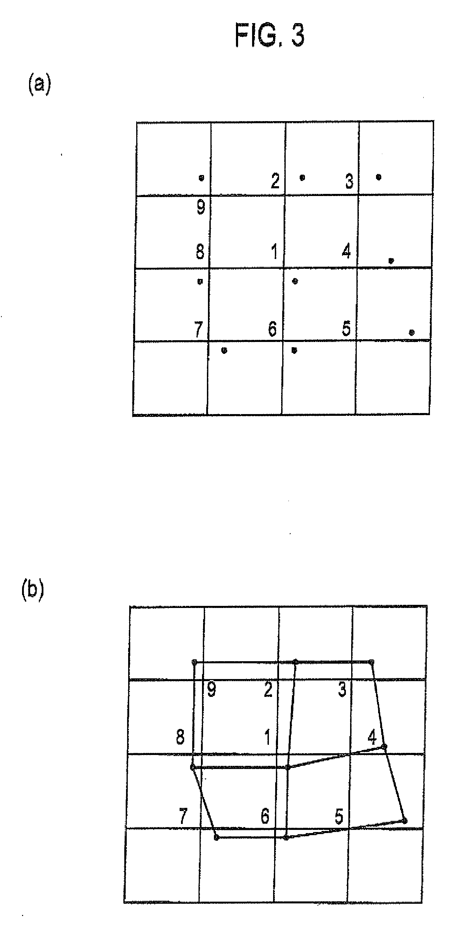

In S30, the output unit 25 outputs display of the examination result chart to a monitor screen or outputs print to the printer, thus terminating the process. The output example of the examination result chart is shown in Fig. 3A. The nine black points in Fig. 3A indicate the positions of the pointer specified by the subject 15 for the points No. 1 to No. 9 on the target screen (targets for fixation). The output unit 25 may display the examination result chart in which the positions of the pointer specified by the subject 15 are connected with line segments.

According to Embodiment 1 described above, the target image generated by the first image generation unit is corrected so as not to look distorted on the screen in the eyes of the subject. This has an effect of providing a natural target image without distortion unlike the conventional image of the grid projected from a fisheye lens.

According to Embodiment 1, the images of the first and second image projection units can be separated by polarizers or the like to be individually presented to the subject's right and left eyes. This has an effect of conducting an ophthalmic examination in a natural condition in which the subject usually lives.

According to Embodiment 1, the output unit presents or outputs the positions of the pointer specified by the subject together with the position of the target image. This has an effect of automatically and accurately recording the eye position deviations without causing an error unlike the case where the examiner copies the eye position deviations to the chart by hand.

### [Embodiment 2]

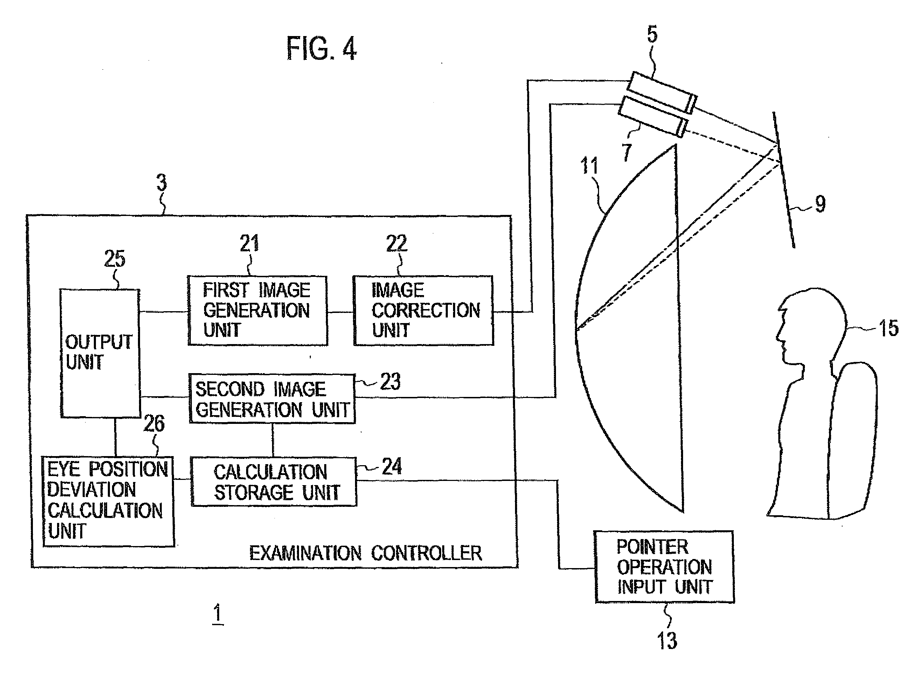

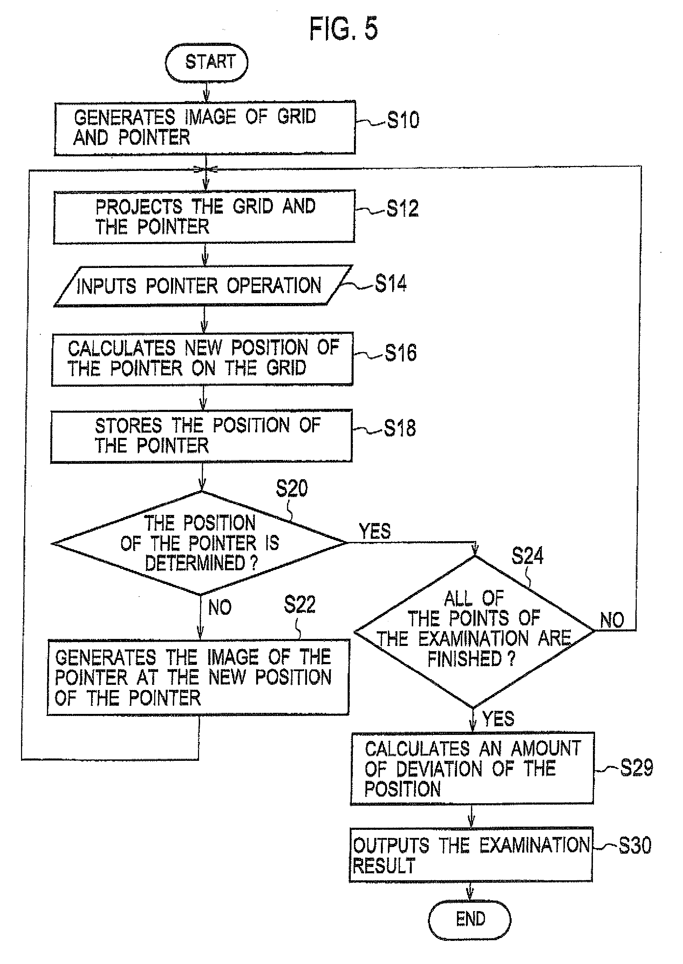

Fig. 4 is a configuration view explaining the configuration of Embodiment 2 of the ophthalmic apparatus according to the invention. Fig. 5 is a flowchart explaining the operation of Embodiment 2.

The ophthalmic apparatus 1 of Embodiment 2 of Fig. 4 further includes an eye position deviation calculation unit 26 within the examination controller 3 in addition to the configuration of Embodiment 1 shown in Fig. 1. The other configuration is the same as that of Fig. 1, and the same constituent components are given the same reference numerals. The redundant description thereof is omitted.

The eye position deviation calculation unit 26 is configured to calculate an amount of eye position deviation of the subject 15 at each examination point based on the position of the pointer calculated and stored by the calculation storage unit 24.

The flowchart of Fig. 5 further includes S29 in addition to the flowchart of Embodiment 1 shown in Fig. 2. If the examination is finished for all the points in the determination of S24, the process proceeds to S29.

In S29, the eye position deviation calculation unit 26 calculates an amount of deviation of the position of the pointer (the indicator target) specified by the subject from the position of the point in the target image (the target for fixation) as angles with respect to the subject's viewing position and outputs the same to the output unit 25.

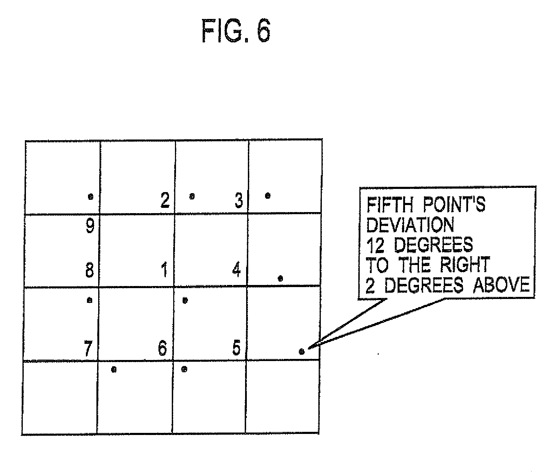

Fig. 6 is a view showing an output example of the calculation result by the eye position deviation calculation unit 26. The drawing shows that, as for the fifth point in the target image (the image for fixation), the amount of deviation of the position of the pointer (the indicator target) specified by the subject is expressed as angles of 12 degrees to the right and 2 degrees above the fifth point. The calculation results can be outputted as results of only coordinates or numerals by respectively setting the right and left deviations to plus and minus values and respectively setting the upward and downward deviations to plus and minus values.

According to Embodiment 2 described above, the target image generated by the first image generation unit is corrected so as not to look distorted on the screen in the eyes of the subject. This has an effect of providing a natural target image without distortion unlike the conventional image of the grid projected from a fisheye lens.

According to Embodiment 2, the images of the first and second image projection units can be separated by polarizers or the like to be individually presented to the subject's right and left eyes. This has an effect of conducting the ophthalmic examination in a natural condition in which the subject usually lives.

According to Embodiment 2, the output unit presents or outputs the position of the pointer specified by the subject together with the position of the target image. This has an effect of automatically and accurately recording the eye position deviations without causing an error unlike the case where the examiner copies the eye position deviations to the chart by hand.

According to Embodiment 2, the eye position deviation calculation unit automatically calculates the amount of deviation of the position of the pointer specified by the subject (the indicator target) from the position of each point in the target image (the target for fixation) as the angles with respect to the subject's viewing position. This has an effect of detecting accurate quantitative deviations.

### [Embodiment 3]

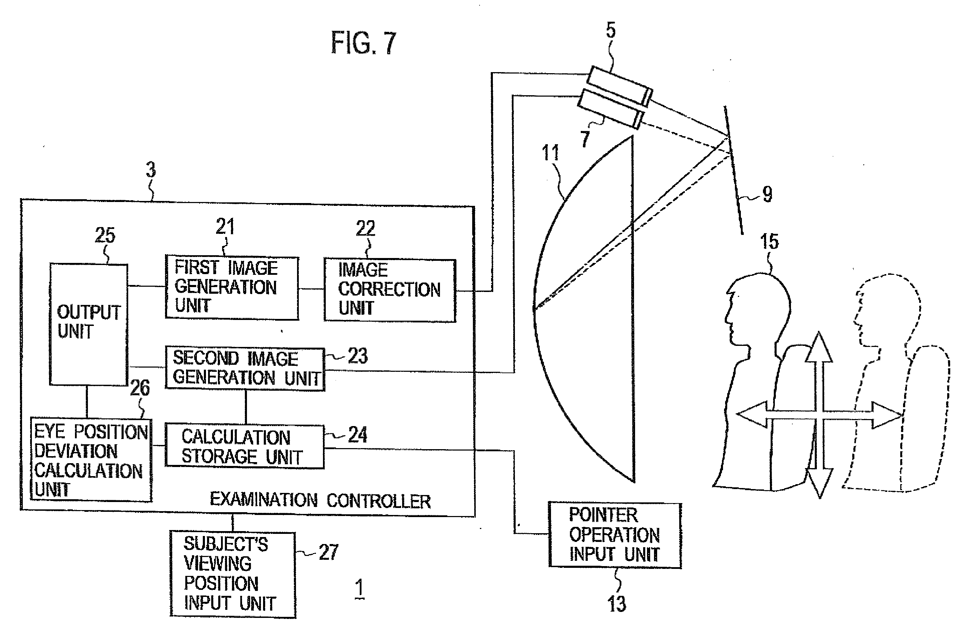

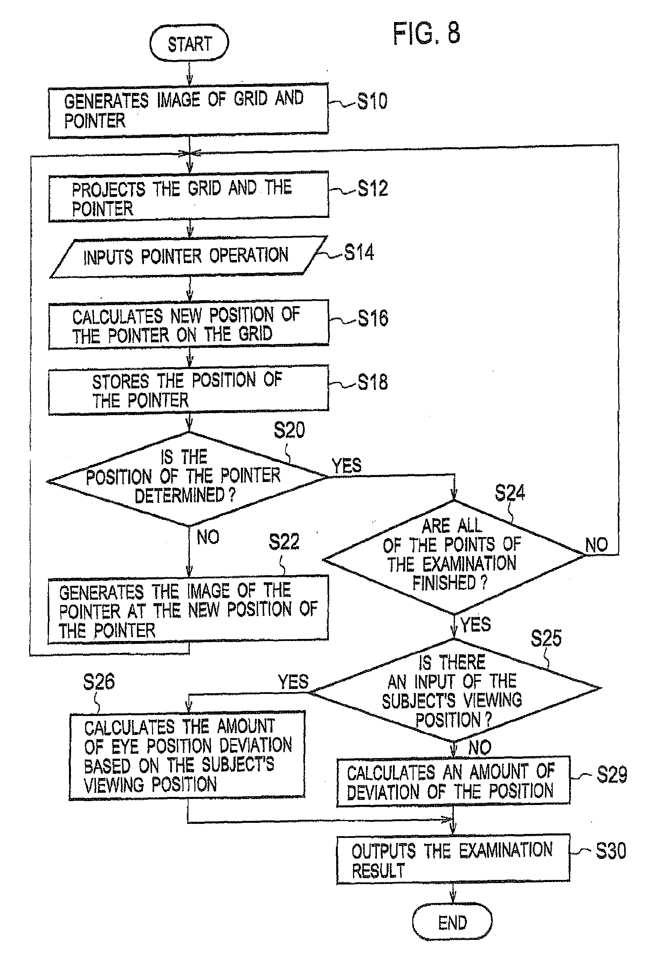

Fig. 7 is a configuration view explaining the configuration of Embodiment 3 of the ophthalmic apparatus according to the invention. Fig. 8 is a flowchart explaining the operation of Embodiment 3.

The ophthalmic apparatus 1 of Embodiment 3 of Fig. 7 includes a subject's viewing position input unit 27 in addition to the configuration of Embodiment 2 shown in Fig. 4. The subject's viewing position input unit 27 is composed of a member by which the examiner manually inputs the subject's viewing position. Alternatively, the subject's viewing position input unit 27 may receive the viewing position from a sensor which is separately provided for the chin rest on which the subject's chin is placed, a chair on which the subject is seated, or the like.

The other configuration is the same as that of Fig. 4, and the same constituent components are given the same reference numerals. The redundant description thereof is omitted. The subject's viewing position input unit 27 is effective especially in the case where the viewing position of the subject 15 cannot be set at a predetermined position with respect to the screen 11. When the screen 11 is spherical, the predetermined position with respect to the screen 11 is the central position of the sphere of the spherical surface thereof.

The flowchart of Fig. 8 includes S25, S26, and S29 in addition to the flowchart of Embodiment 1 shown in Fig. 2. The other steps are the same as those of Fig. 2, and only the added steps are described. If the examination for all the points is finished in the determination in S24, the process proceeds to S25.

In S25, it is determined whether there is an input of the subject's viewing position from the subject's viewing position input unit 27. If there is an input of the subject's viewing position, the process proceeds to S26, and if there is no input of the subject's viewing position, the process proceeds to S29.

In S26, based on the input of the subject's viewing position from the subject's viewing position input unit 27 and the examination results, the eye position deviation calculation unit 26 calculates the amount of eye position deviation and outputs the same to the output unit 25.

In S29, similarly to Embodiment 2, the eye position deviation calculation unit 26 calculates the amount of deviation of the position of the pointer (the indicator target) specified by the subject from the position of each point in the target image (the target for fixation) as angles with respect to the subject's viewing position. The eye position deviation calculation unit 26 then outputs the calculated amount of deviation to the output unit 25.

According to Embodiment 3 described above, the target image generated by the first image generation unit is corrected so as not to look distorted on the screen in the eyes of the subject. This has an effect of providing a natural target image without distortion unlike the conventional image of the grid projected from a fisheye lens.

According to Embodiment 3, the images of the first and second image projection units can be separated by polarizers or the like to be individually presented to the subject's right and left eyes. This has an effect of conducting the ophthalmic examination in a natural condition in which the subject usually lives.

According to Embodiment 3, the output unit presents or outputs the position of the pointer specified by the subject together with the position of the target image. This has an effect of automatically and accurately recording the eye position deviations without causing an error unlike the case where the examiner copies the eye position deviations to the chart by hand.

According to Embodiment 3, the eye position deviation calculation unit automatically calculates the amount of deviation of the position of the pointer specified by the subject (the indicator target) from the position of each point in the target image (the target for fixation) as the angles with respect to the subject's viewing position. This has an effect of detecting accurate quantitative deviations.

According to Embodiment 3, there is an effect of providing quantitatively accurate examination results by causing the examiner to input the subject's viewing position even if the viewing position of the subject 15 cannot be located at a predetermined position with respect to the screen 11.

### [Embodiment 4]

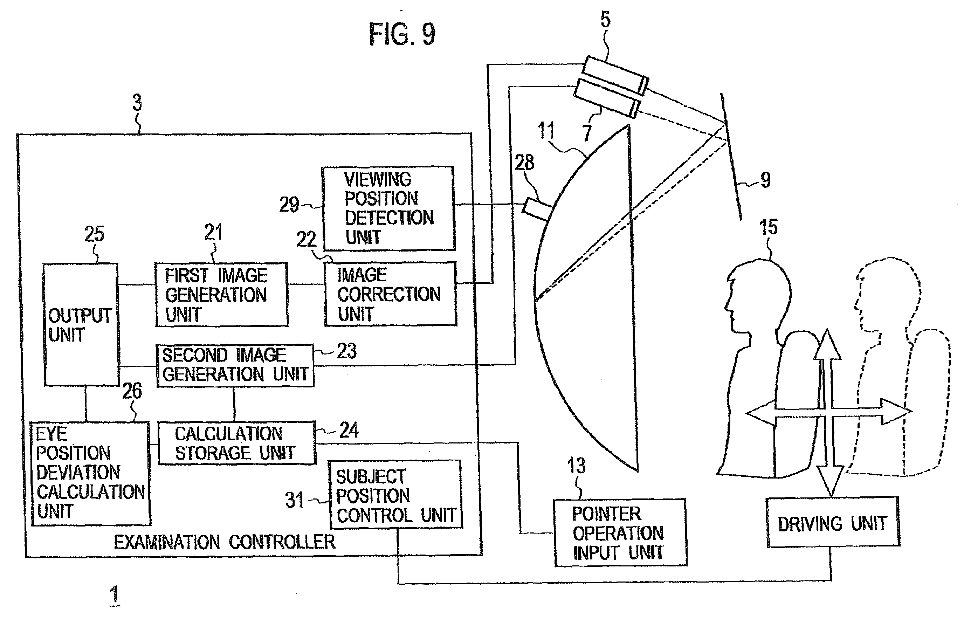

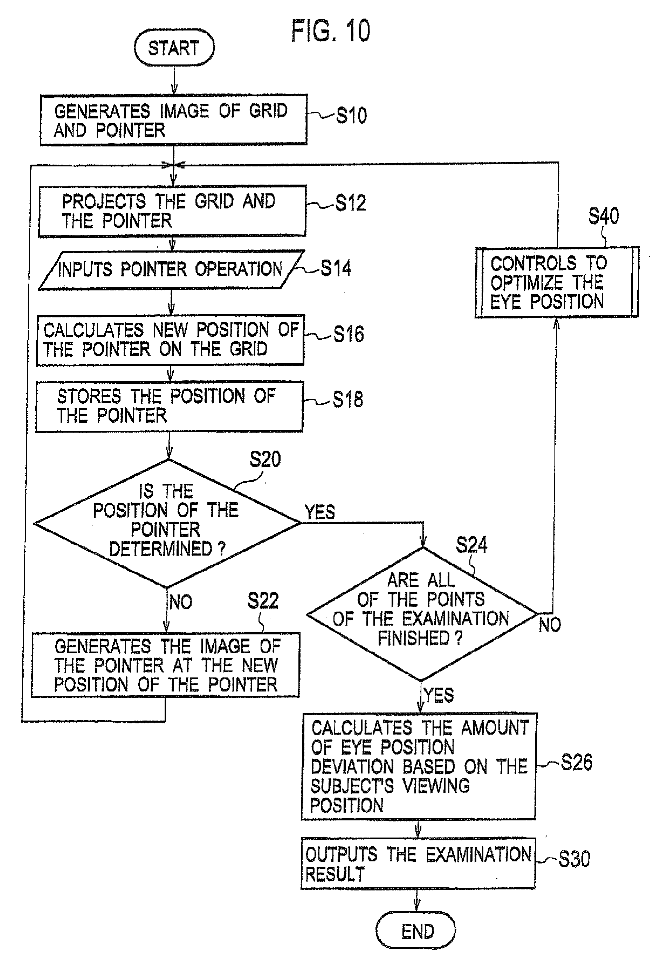

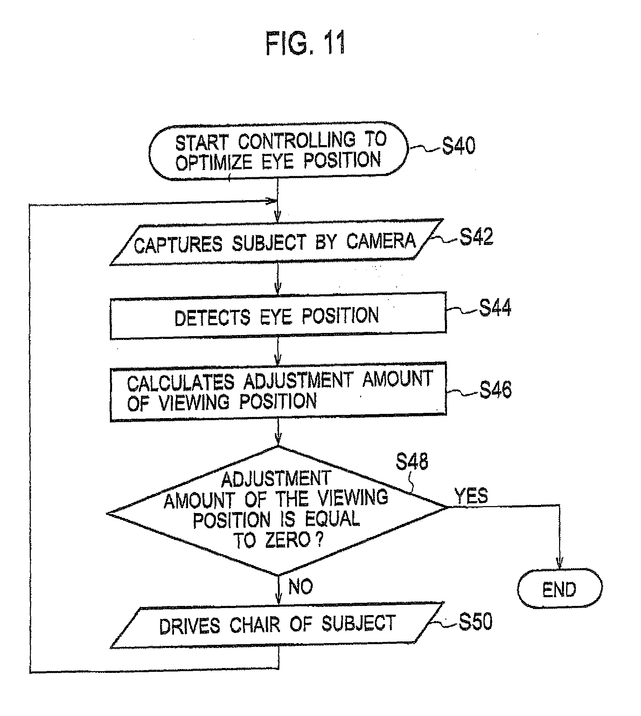

Fig. 9 is a configuration view explaining the configuration of Embodiment 4 of the ophthalmic apparatus according to the invention. Figs. 10 and 11 are flowcharts explaining the operation of Embodiment 4.

In addition to the configuration of Embodiment 2 shown in Fig. 4, the ophthalmic apparatus 1 of Embodiment 4 of Fig. 9 includes: a camera 28 capturing an image of the face of the subject 15; a viewing position detection unit 29 detecting the eye position of the subject 15 based on the image of the face captured by the camera 28 to detect the viewing position; a driving unit 30 moving the subject's position; and a subject position control unit 31 controlling the driving unit 30 based on the detection results by the viewing position detection unit 29 to operate the position of the subject 15.

The viewing position detection unit 29 and the subject position control unit 31 are provided within the examination controller 3. The other configuration is the same as that of Fig. 4, and the same constituent components are given the same reference numerals. The redundant description thereof is omitted.

The camera 28 is a camera capturing an image of the face of the subject 15 such as a visible camera, an infrared camera, or the like. In order to detect the viewing position of the subject 15 from the image of the face captured by the camera 28, it is preferable to capture images of the face of the subject 15 in plural directions by providing a plurality of the cameras 28 or causing the camera 28 to move along a rail provided at the periphery of the screen 11. The viewing position detection unit 29 detects the viewing position of the subject 15 based on the image of the face of the subject 15 captured by the camera 28.

The driving unit 30 drives the chin rest on which the chin of the subject 15 is placed or drives the chair of the subject 15 to operate the position of the subject 15. Alternatively, instead of moving the subject 15, the driving unit 30 may be configured to drive a part of the ophthalmic apparatus 1 including the first and second image projection units 5 and 7, mirror 9, screen 11, and camera 28.

The drive control unit 31 controls the driving unit 30 to always optimally control the position of the subject 15 with respect to the screen 11 based on the viewing position of the subject 15 detected by the viewing position detection unit 29.

The flowchart of Fig. 10 includes S26 and S40 in addition to the flowchart of Embodiment 1 shown in Fig. 2. Fig. 11 is a detailed flowchart explaining the details of S40. The other steps are the same as those of Fig. 2, and only the added steps are described. If the examination for all the points is finished in the determination in S24, the process proceeds to S26. If the examination for all the points is not finished in the determination in S24, the process proceeds to S40.

In S26, based on the information about the viewing position detected by the viewing position detection unit 29, the eye position deviation calculation unit 26 calculates the amount of eye position deviation of the examination results and outputs the same to the output unit 25.

In S40, the subject position control unit 31 controls the driving unit 30 to adjust and optimize the eye position of the subject 15.

Next, with reference to Fig. 11, the optimal adjustment of the viewing position of the subject 15 in S40 is described in detail.

If the examination for all the examination points is finished in S24, the process proceeds to S40, and optimal adjustment of the viewing position is started.

In S42, first, the image of the face of the subject 15 is captured by the camera 28.

Next, in S44, the viewing position detection unit 29 detects the eye position from the image of the face of the subject 15 to detect the viewing position.

Next, in S46, the subject position control unit 31 calculates an adjustment amount of the viewing position which is a difference between the viewing position detected by the viewing position detection unit 29 and a predetermined optimal viewing position (which is normally the center of the screen or may be any different position).

Next, in S48, it is determined whether the adjustment amount of the viewing position is equal to zero. If the adjustment amount of the viewing position is equal to zero, the process is terminated.

If the adjustment amount of the viewing position is not equal to zero, the process proceeds to S50.

In S50,the driving unit 30 drives the chair of the subject 15 up, down, rightward, leftward, forward, and rearward. The drive amount of the chair can be calculated with reference to a map controlling the drive amount of the chair with respect to previously stored adjustment amounts of the viewing position. If the drive in S50 is finished, the process returns to S42, and the image of the subject 15 is captured by the camera 28 again. If the adjustment amount in S48 reaches zero after repetition of the capture of the image of the subject 15 and the drive of the chair, the optimal adjustment of the viewing position is terminated.

According to Embodiment 4 described above, the target image generated by the first image generation unit is corrected so as not to look distorted on the screen in the eyes of the subject. This has an effect of providing a natural target image without distortion unlike the conventional image of the grid projected from a fisheye lens.

According to Embodiment 4, the images of the first and second image projection units can be separated by polarizers or the like to be individually presented to the subject's right and left eyes. This has an effect of conducting the ophthalmic examination in a natural condition in which the subject usually lives.

According to Embodiment 4, the output unit presents or outputs the positions of the pointer specified by the subject together with the position of the target image. This has an effect of automatically and accurately recording the eye position deviations without causing an error unlike the case where the examiner copies the eye position deviations to the chart by hand.

According to Embodiment 4, the eye position deviation calculation unit automatically calculates the amount of deviation of the position of the pointer specified by the subject (the indicator target) from the position of each point in the target image (the target for fixation) as the angles with respect to the subject's viewing position. This has an effect of detecting accurate quantitative deviations.

According to Embodiment 4, there is an effect of providing quantitatively accurate examination results by causing the examiner to input the subject's viewing position even if the viewing position of the subject 15 cannot be located at a predetermined position with respect to the screen 11.

According to Embodiment 4, the subject's viewing position detected by the viewing position detection unit can be optimized by drive of the driving unit, and it is possible to keep the region for the examination object maximized.

As a modification of Embodiment 4, only the camera 28 and viewing position detection unit 29 may be added to Embodiment 2. This has an effect of, in addition to the effects of Embodiment 2, providing quantitatively accurate examination results based on the subject's viewing position detected by the viewing position detection unit even if the viewing position of the subject 15 cannot be located at the predetermined position with respect to the screen 11.

The preferred embodiments are described above but do not limit the present invention. It is obvious that various changes can be made according to the design and the like without departing from the technical idea according to the invention other than the aforementioned embodiments.

### [Industrial Applicability]

The present invention is applicable to the industries manufacturing and selling devices used in the ophthalmic field.

### Explanation of Reference Numerals

- 1:: OPHTHALMIC APPARATUS
- 3;: EXAMINATION CONTROLLER
- 5:: FIRST IMAGE PROJECTION UNIT
- 7:: SECOND IMAGE PROJECTION UNIT
- 9:: MIRROR
- 11:: SCREEN
- 13:: POINTER OPERATION INPUT UNIT
- 15:: SUBJECT
- 21:: FIRST IMAGE GENERATION UNIT
- 22:: IMAGE CORRECTION UNIT
- 23:: SECOND IMAGE GENERATION UNIT
- 24:: CALCULATION STORAGE UNIT
- 25:: OUTPUT UNIT
- 26:: EYE POSITION DEVIATION CALCULATION UNIT
- 27:: SUBJECT'S VIEWING POSITION INPUT UNIT
- 28:: CAMERA
- 29:: VIEWING POSITION DETECTION UNIT
- 30:: DRIVING UNIT
- 31:: SUBJECT POSITION CONTROL UNIT

## Claims

1. An ophthalmic apparatus comprising:
a screen keeping distance constant from a subject's eye position;
a first image generation unit generating a target image;
an image correction unit correcting the target image generated by the first image generation unit to cause the target image not to look distorted on the screen in the eyes of the subject;
a first image projection unit projecting onto the screen, the target image generated by the first image generation unit;
a pointer operation input unit through which the subject inputs the positional operation of the pointer;
a calculation storage unit calculating the position of the pointer in the target image according to the input through the pointer operation input unit and storing the calculated position;
a second image generation unit generating the image of the pointer at the position in the target image calculated by the calculation storage unit;
a second image projection unit projecting onto the screen, the image generated by the second image generation unit; and
an output unit presenting or outputting the position of the pointer together with the target image.

2. The ophthalmic apparatus according to claim 1, further comprising an eye position deviation calculation unit converting the position of the pointer in the target image calculated by the calculation storage unit to a deviation angle with respect to a viewing position of the subject and outputting the deviation angle to the output unit.

3. The ophthalmic apparatus according to claim 2, further comprising a subject's viewing position input unit through which an examiner inputs the eye position of the subj ect.

4. The ophthalmic apparatus according to claim 2, further comprising a subject's viewing position detection unit detecting the eye position of the subject based on an image obtained by capturing an image of the subject.

5. The ophthalmic apparatus according to claim 4, further comprising:
a driving unit moving the position of the subject; and
a subject position control unit controlling the driving unit based on results of the detection by the subject's viewing position detection unit to operate the position of the subject with respect to the screen.

6. A Hess test apparatus comprising the ophthalmic apparatus according to any one of claims 1 to 5.
